(19) Europäisches Patentamt
European Patent Office
Office européen des brevets

(11) **EP 3 808 258 A1**

(12) **EUROPEAN PATENT APPLICATION**
published in accordance with Art. 153(4) EPC

(43) Date of publication:
**21.04.2021 Bulletin 2021/16**

(51) Int Cl.:
**A61B 5/02** (2006.01)

(21) Application number: **18798595.7**

(22) Date of filing: **06.06.2018**

(86) International application number:
**PCT/CN2018/090089**

(87) International publication number:
**WO 2018/206014 (15.11.2018 Gazette 2018/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Hangzhou Megasens Technologies Co., Ltd.**
**Hangzhou, Zhejiang 310052 (CN)**

(72) Inventors:
• ZHOU, Congcong
Hangzhou
Zhejiang 310052 (CN)
• YUAN, Haiquan
Hangzhou
Zhejiang 310052 (CN)

(74) Representative: **Dr. Weitzel & Partner**
**Patent- und Rechtsanwälte mbB**
**Friedenstrasse 10**
**89522 Heidenheim (DE)**

(54) **DUAL OPTICAL PATH SENSOR MODULE**

(57) The present invention discloses a dual optical path sensor module for collect human physiological signals based on photoplethysmography. The module includes a base, and a photoelectric receiver and two light source modules mounted on the base. The two light source modules are symmetrically disposed on two sides of the photoelectric receiver, and each light source module includes light sources with at least two wavelengths. A light shielding portion is further disposed on the base, the light shielding portion surrounds the photoelectric receiver, and there is an opening at the top of the light shielding portion, so that light emitted from the light source is received by the photoelectric receiver after the light is diffusely reflected by the human body tissue.

FIG. 1

**Description**

**TECHNICAL FIELD**

The present invention discloses a dual optical path sensor module, relates to the field of photoelectric physiological signal collection, and can be applied to various places requiring long-term monitoring of blood oxygen saturation and heart rate.

**BACKGROUND**

The human pulse wave contains rich physiological information, and can provide a basis for evaluating the physical state of the individual by collecting and analyzing the pulse wave, on the premise of that the pulse wave signal can be obtained stably and reliably. For example, pulse blood oxygen saturation reflects the amount of oxygen in the blood. Like the pulse rate, it is a basic human physiological parameter and plays an important role in clinical diagnostic analysis. The non-invasive blood oxygen saturation monitoring method is generally based on photoplethysmography, and light sources with at least two different wavelengths are required for excitation.

Currently, the common monitoring devices on the market are heavy in load and poor in wearing comfort, so it is difficult to meet the requirement of long-term continuous pulse wave oxygen saturation and heart rate monitoring. Domestic researches, such as CN201585990 and CN102961144, attempt to detect blood oxygen saturation on the finger and designs are made therein. Currently, there are also related conceptual products on the market, in which the principle adopted is basically based on transmissive oxygen saturation detection, which is easy to be interfered by motion and difficult to achieve accurate physiological parameter over-limit alarm prompts and local real-time feedback. In CN204520642U, two photoelectric receiving tubes are used to reduce the influence of dark current, but signal instability caused by shift cannot be avoided.

To solve the above problem, a dual optical path sensor module is designed, and it is necessary to stably obtain the physiological signal.

**SUMMARY**

For the shortcomings existing in the prior art, a dual optical path sensor module is designed in the present invention, and the module includes a photoelectric receiver and two pairs of symmetrical light sources, so that stability of signal quality can be improved.

Human tissue is a high scatterer, and the incident light travels in a "banana-shaped" path within a uniform medium. Photons are partially absorbed by tissues in human tissues, and are also scattered by the tissues and spread in the tissues. Assuming that the human tissue is uniform and homogeneous, the human issue can usually be divided into the following four parts: arteries, capillaries, veins, and blood-free parts such as bones and fats, and blood in the arteries and the capillaries and blood in the tissues are different in optical properties. When the heart contracts, the absorption and scattering coefficients in the arterial blood vessels and capillaries increase, resulting in a decrease in the luminous flux density received by the photoelectric receiving tube.

From the Lambert-Beer law, the light intensity produced by the diffuse reflection of the tissue and received at the receiving tube can be expressed by:

$$P_{pd}(t) \propto [\beta_c - (1 + \frac{\Delta l_{bv}(t)}{l_{bv}})\beta_1 \Delta c_b(t) - \beta_2 \Delta l_{ed}(t) - \beta_3 \Delta l_{bv}(t)] \tag{1-1}$$

In the formula (1-1), $\beta_c$, $\beta_1$, $\beta_2$, $\beta_3$ are scale coefficients obtained by Lambert Beer's first-order Taylor expansion, and $\Delta l_{ed}(t)$ and $\Delta l_{bv}(t)$ are optical path changes caused by motion. It can be seen from the formula that body motion causes a change in the optical path, thereby causing a change in the light intensity at the receiving tube. In addition, $\beta_1 \Delta c_b(t)$ is dispersed by $\frac{\Delta l_{bv}(t)}{l_{bv}}$ through motion. In this case, the change in the optical path is mainly caused by the change in the distance between the light-emitting tube and the receiving tube, and the deformation of the tissue.

A dual optical path sensor module designed in the present invention is applicable to collecting human physiological signals based on photoplethysmography. The module includes a base, and a photoelectric receiver and two light source modules mounted on the base. The two light source modules are symmetrically disposed on two sides of the photoelectric receiver, and each light source module includes light sources with at least two wavelengths. A light shielding portion is further disposed on the base, the light shielding proton surrounds the photoelectric receiver, and there is an opening at the top of the light shielding portion, so that light emitted from the light source is received by the photoelectric receiver after the light is diffusely reflected by the human body tissue.

The light source with a short wavelength in each light source module is closer to the photoelectric receiver.

Further, the light shielding portion is made of an elastic material.

The dual optical path sensor module designed in the present invention can obtain the physiological signals more stably. The module can continuously monitor pulse wave blood oxygen saturation and heart rate for a long time, has a strong anti-interference ability, and has broad application prospects.

## BRIEF DESCRIPTION OF DRAWINGS

FIG. 1 is a top view according to an embodiment of the present invention.
FIG. 2 is a front view according to an embodiment of the present invention.
FIG. 3 is a schematic diagram of an optical path of an embodiment of the module on the skin surface.

## DESCRIPTION OF EMBODIMENTS

To make technical means, creative features, achievements of purpose and effect implemented by the present invention easy to understand, the present invention is further described with reference to the accompanying drawings and specific embodiments.

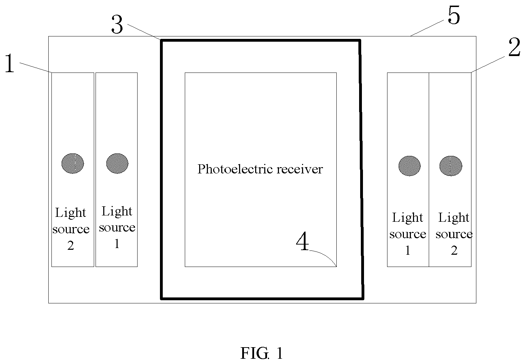

Referring to FIG. 1, the following technical solution is used according to an embodiment of the present invention: A dual optical path sensor module includes a photoelectric receiver 4 and two light source modules 1 and 2. There is a light shielding portion 3 between the photoelectric receiver 4 and the light source modules 1 and 2. The light source modules 1 and 2 each include light sources with at least two wavelengths: a light source 1 and a light source 2 respectively. The light sources can be extended according to needs. A wavelength of the light source 1 is shorter than a wavelength of the light source 2 in the light source modules 1 and 2. The light source 1 is closer to the photoelectric receiving tube. When the light sources are extended, the light source with a short wavelength is closer to the photoelectric receiver. The light source modules 1 and 2 are separately located on two sides of the photoelectric receiver 4, and are spatially symmetric. Light sources with different wavelengths are diffusely reflected by the tissue and then reach the photoelectric receiver 4.

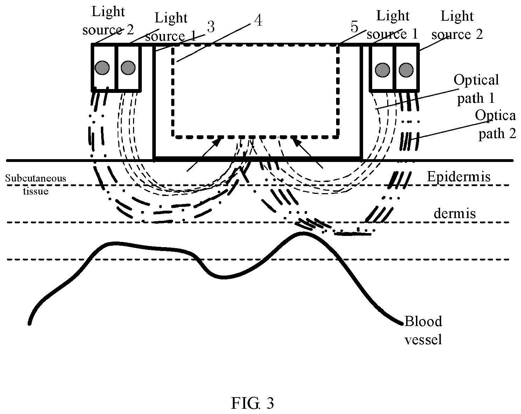

FIG. 2 is a front view according to an embodiment of the present invention. The light shielding portion 3 surrounds the photoelectric receiver 4, there is an opening at the top of the light shielding portion 3, and lights emitted from the light source modules 1 and 2 cannot directly enter the photoelectric receiver 4. In this design, the interference introduced by light leakage can be effectively avoided, and the light shielding portion 3 results in that the light emitted from the two light source modules 1 and 2 needs to be diffusely reflected before reaching the photoelectric receiver 4.

FIG. 3 is a schematic diagram of an optical path of an embodiment of the module on the skin surface. The light emitted from the light source 1 and the light source 2 has the properties of reflection, projection and refraction. In this module, the light source 1 generates an effective optical path, and the optical path 1 is taken as an example. The light source 2 generates an effective optical path, and the optical path 2 is taken as an example. A photon is diffusely reflected through epidermis, dermis, and subcutaneous tissue, and then reaches the photoelectric receiver 4. The photon cannot directly penetrate the light shielding portion 3 to directly reach the photoelectric receiver 4. Considering that the module is placed on a human body for use, the light shielding portion 3 can be made of an elastic material.

The purpose and effect achieved by the present invention are expressed by the formula (1-1). For the dual optical path sensor module designed in the present invention, the light source modules 1 and 2 are separately located on two sides of the photoelectric receiving tube 4, and are symmetrically distributed. And the wavelength of the light source 1 is shorter than that of the light source 2. The interference caused by the shift can be suppressed by the optical path to some extent. There is a light shielding portion 3 between the photoelectric receiving tube 4 and the light source modules 1 and 2, so that the interference introduced by light leakage can be effectively avoided. The light shielding portion 3 is not limited to the one shown in the drawings. To effectively avoid the interference introduced by light leakage, any structure that can block the light of the light source that is directly lighted to the photoelectric receiving tube is possible.

The distance between the light source 1 and the photoelectric receiving tube 4 and the distance between the light source 2 and the photoelectric receiving tube 4 can theoretically be described by the following method. The human skin tissue is approximated as a semi-infinite medium. A diffusely reflected light detected far from an incident point d is analyzed. An xz coordinate system is established. The incident point is taken as an origin, and a line connecting the incident point to a detection point is an x-axis, and a light normal-incidence direction is a z-axis. The coordinates of the incident point are (0,0,0), and the coordinates of a receiving point are (d,0,0). In the study of this text, a light-emitting diode LED is located at the coordinates of the incident point, the intended receiving point to be placed is at the location of the exiting light, and the coordinate of it is (d, 0, 0). According to the definition of the curve, the coordinate value of the z-axis is defined as a function of x: $z_0(x)$:

$$z_0(x) \approx \sqrt{\frac{2\mathrm{x}(d-x)}{\sqrt{3\mu_a\mu_s}d}} \quad (1\text{-}2).$$

According to the characteristics of the "banana-shaped" path, it can be derived that there is a maximum value or a minimum value for the z-axis coordinate of the curve at a position of $\mathrm{x} = \dfrac{d}{2}$, and the value can be obtained according to the formula (1-3):

$$z_0(x)_{\max} \approx \sqrt{\frac{d}{2\sqrt{3}\mu_a\mu_s}} \quad (1\text{-}3),$$

Wherein, $\mu_a$ is the absorption coefficient of the medium, $\mu_s$ is the reduced scattering coefficient of the medium. It can be seen from the formula (1-3) that, there is a certain relationship between the value of the radial distance and the penetration depth of the skin. Human skin tissue has an obvious layered structure and is divided into three layers from the surface to the inside: the epidermis layer, the dermis layer, and the subcutaneous tissue layer. Because of the thicker dermis layer in the skin tissue, the near-infrared light that penetrates the dermis layer into the subcutaneous tissue largely attenuates, and the diffusely reflected light energy that can be returned can be approximately neglected. Assuming that the steady-state light transmission probability density function is $\rho(\rho,z)$, the thickness of the skin layer and the thickness of the dermis layer are respectively $dep_1$ and $dep_2$, and the proportion of corresponding layers can be obtained as follows:

$$f_1(\rho) = \frac{\int_0^{dep_1} \rho(\rho,z)\,dz}{\int_0^{\infty} \rho(\rho,z)\,dz} \quad (1\text{-}4)$$

$$f_2(\rho) = \frac{\int_{dep_1}^{dep_1+dep_2} \rho(\rho,z)\,dz}{\int_0^{\infty} \rho(\rho,z)\,dz} \quad (1\text{-}5)$$

Assuming that the diffuse probability of light in each layer of medium is averagely distributed, the average value of d can be calculated from the formula (1-6):

$$\mathrm{d}_{ave} = \frac{1}{3}\sum_{i=1}^{3} d(i) \times z(i) \quad (1\text{-}6)$$

There may be a theoretical prediction of the value of the radial distance according to the formula (1-6).

The basic principles and main features of the present invention and the advantages of the present invention are shown and described above. It should be understood by persons skilled in the art that the present invention is not limited by the foregoing embodiments, and the foregoing embodiments and the description of the present invention are just for illustrating the principles of the present invention. Without departing from the spirit and scope of the present invention, various changes and improvements are intended to fall within the protection scope of the present invention. The protection scope of the present invention is defined by the appended claims and their equivalents.

**Claims**

1. A dual optical path sensor module for collect human physiological signals based on photoplethysmography, wherein the module comprises a base, and a photoelectric receiver and two light source modules mounted on the base, the two light source modules are symmetrically disposed on two sides of the photoelectric receiver, and each light source module comprises light sources with at least two wavelengths; a light shielding portion is further disposed on the base, the light shielding portion surrounds the photoelectric receiver, and there is an opening at the top of the light shielding portion, so that light emitted from the light source is received by the photoelectric receiver after the light is diffusely reflected by the human body tissue.

2. The dual optical path sensor module according to claim 1, wherein the light source with a short wavelength in each light source module is closer to the photoelectric receiver.

3. The dual optical path sensor module according to claim 1, wherein the light shielding portion is made of an elastic material.

FIG. 1

FIG. 3

## INTERNATIONAL SEARCH REPORT

| International application No. |
|---|
| PCT/CN2018/090089 |

**A. CLASSIFICATION OF SUBJECT MATTER**

A61B 5/02 (2006.01) i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNABS; CNTXT; VEN; USTXT; EPTXT; WOTXT; CNKI: 脉搏, 双光路, 传感, 光源, 发光, 激光, 光电, 光敏, 波长, 接收, 发射, light+, LED, emit+, receiv+, wavelength, sens+

**C. DOCUMENTS CONSIDERED TO BE RELEVANT**

| Category* | Citation of document, with indication, where appropriate, of the relevant passages | Relevant to claim No. |
|---|---|---|
| Y | JP 2016002167 A (GENIAL LIGHT CO., LTD.), 12 January 2016 (12.01.2016), description, paragraphs [0018]-[0059], and figures 1-6 | 1-3 |
| Y | CN 106308753 A (MA, Dongge), 11 January 2017 (11.01.2017), description, paragraphs [0044]-[0066], and figure 1 | 1-3 |
| A | US 2016220133 A1 (SEIKO EPSON CORP.), 04 August 2016 (04.08.2016), entire document | 1-3 |
| A | JP 2017006183 A (PANASONIC IP MAN CORP.), 12 January 2017 (12.01.2017), entire document | 1-3 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 27 June 2018 | 06 August 2018 |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer ZOU, Pan Telephone No. 86-(512)-88997373 |
|---|---|

Form PCT/ISA/210 (second sheet) (January 2015)

## INTERNATIONAL SEARCH REPORT
### Information on patent family members

| International application No. |
|---|
| PCT/CN2018/090089 |

| Patent Documents referred in the Report | Publication Date | Patent Family | Publication Date |
|---|---|---|---|
| JP 2016002167 A | 12 January 2016 | None | |
| CN 106308753 A | 11 January 2017 | None | |
| US 2016220133 A1 | 04 August 2016 | JP 2016140642 A | 08 August 2016 |
| JP 2017006183 A | 12 January 2017 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201585990
- CN 102961144
- CN 204520642 U